Europäisches Patentamt

European Patent Office

Office européen des brevets

(11) Veröffentlichungsnummer: **0 338 366**
**A1**

(12) **EUROPÄISCHE PATENTANMELDUNG**

(21) Anmeldenummer: 89106284.6

(22) Anmeldetag: 10.04.89

(51) Int. Cl.⁴: **C07C 143/02**

(30) Priorität: 18.04.88 DE 3812846

(43) Veröffentlichungstag der Anmeldung:
25.10.89 Patentblatt 89/43

(84) Benannte Vertragsstaaten:
AT BE CH DE ES FR GB GR IT LI NL SE

(71) Anmelder: Henkel Kommanditgesellschaft auf Aktien
Postfach 1100 Henkelstrasse 67
D-4000 Düsseldorf 1(DE)

(72) Erfinder: Bunte, Reinhard, Dr.
Sulzbastrasse 69
D-4000 Düsseldorf 12(DE)
Erfinder: Beuer, Bernd
Grazer Strasse 43
D-4019 Monheim 2(DE)

(54) Verfahren zur Herstellung von C1-C6-Alkansulfonsäuren.

(57) Geradkettige oder verzweigte $C_1$-$C_6$-Alkansulfonsäuren lassen sich aus den Alkalisalzen derselben herstellen, indem man die Alkalisalze in einer Lösung oder Suspension in $C_1$-$C_4$-Monoalkanolen mit Chlorwasserstoff umsetzt, die ausgefallenen Alkalichloride abtrennt und die $C_1$-$C_6$-Alkansulfonsäuren aus der $C_1$-$C_4$-Monoalkanolphase isoliert.

EP 0 338 366 A1

## Verfahren zur Herstellung von $C_1$-$C_6$-Alkansulfonsäuren

Die Erfindung betrifft ein Verfahren zur Herstellung geradkettiger oder verzweigter $C_1$-$C_6$-Alkansulfonsäuren aus den Alkalisalzen derselben.

Kurzkettige 1-Sulfonsäuren sind als Substitut für Phosphorsäure in phosphatfreien Reinigungsmitteln vorgeschlagen worden, stehen zur Zeit jedoch nicht in großtechnischen Mengen zur Verfügung.

Kurzkettige Alkansulfonsäuren können durch Oxidation der entsprechenden Mercaptane mit Salpetersäure oder Persäuren sowie mit Jod in Gegenwart von Bromidionen in Dimethyl-sulfoxid hergestellt werden. Diese Verfahren ergeben jedoch nur mäßige Ausbeuten. Zudem sind Mercaptane für großtechnische Verfahren zu teuer.

Kurzkettige Alkansulfonsäuren lassen sich weiterhin aus Alkanen durch Sulfochlorierung mit anschließender Verseifung des Alkansulfonylchlorids oder durch Sulfoxidation herstellen; die Sulfonierung erfolgt jedoch an der Kohlenwasserstoffkette in statistischer Verteilung und zudem auch gegebenenfalls mehrfach, so daß die selektive Herstellung von 1-Sulfonsäuren nicht möglich ist.

Auch dem Umsetzung von Olefinen mit Natriumhydrogensulfit und anschließender Freisetzung der Alkansulfonsäuren ergibt unerwünschte Nebenprodukte.

Aus Bl.Chem.Soc. Japan 32, 850(1959) ist die Umsetzung von kurzkettigen Alkylhalogeniden mit Natriumsulfit zu den entsprechenden Alkylnatriumsulfonaten nach der sogenannten Strecker-Synthese bekannt; die Freisetzung der kurzkettigen Alkansulfonsäuren aus den erhaltenen Natriumsalzen ist jedoch nicht beschrieben. Sie stößt auch auf Schwierigkeiten, weil sowohl die kurzkettigen Alkansulfonsäuren als auch die Salze derselben in Wasser gut löslich sind.

Die Erfindung ist somit auf ein Verfahren zur Herstellung geradkettiger oder verzweigter $C_1$-$C_6$-Alkansulfonsäuren gerichtet, das sich im großtechnischen Maßstab durchführen läßt und die gewünschten Endprodukte in großer Reinheit ergibt.

Diese Aufgabe wird erfindungsgemäß durch ein Verfahren der eingangs genannten Art gelöst, bei dem man die Alkalisalze in einer Lösung oder Suspension von $C_1$-$C_4$-Monoalkanolen mit Chlorwasserstoff umsetzt, die ausgefallenen Alkalichloride abtrennt und die $C_1$-$C_6$-Alkansulfonsäuren aus der $C_1$-$C_4$-Monoalkanolphase isoliert.

Mit dem Verfahren der Erfindung können insbesondere 1-Alkansulfonsäuren der angegebenen Kettenlänge hergestellt werden; sie können praktisch isomerenfrei erhalten werden, wenn man von den entsprechenden isomerenfreien Alkalisulfonaten ausgeht.

Zweckmäßigerweise führt man das Verfahren der Erfindung in der Weise durch, daß man zunächst die Alkalisalze in $C_1$-$C_4$-Monoalkanolen löst oder vorzugsweise suspendiert und anschließend gasförmigen Chlorwasserstoff in die Lösung oder Suspension einleitet; es ist jedoch auch möglich, eine Lösung von gasförmigen Chlorwasserstoff in Monoalkanolen vorzulegen und die Alkalisalze einzutragen. Nach Abtrennung der im Verlauf der Reaktion ausfallenden Alkalichloride, z.B. durch Filtration oder Zentrifugieren, können die gebildeten $C_1$-$C_6$-Alkansulfonsäuren in an sich bekannter Weise isoliert werden, z.B. durch Abdampfen der $C_1$-$C_4$-Monoalkanole.

Als $C_1$-$C_4$-Monoalkanole können, Methanol, Ethanol, n-Propanol, i-Propanol, n-Butanol, sec-Butanol oder tert.-Butanol sowie Mischungen derselben eingesetzt werden; die Verwendung von Methanol ist bevorzugt. Weiterhin verwendet man bevorzugt die Natriumsalze der $C_1$-$C_6$-Alkansulfonsäuren als Ausgangsmaterial.

Gemäß einer weiteren vorteilhaften Ausführungsform der Erfindung verwendet man die Natriumsalze der $C_1$-$C_6$-Alkansulfonsäuren in Form von Gemischen mit Natriumhalogeniden, wie sie in an sich bekannter Weise durch Umsetzung der entsprechenden $C_1$-$C_6$-Alkylhalogenide mit Natriumsulfit in wässrigen Lösungen und Eindampfen des Reaktionsgemisches erhältlich sind. Eine Abtrennung der Alkansulfonsäuresalze von den Natriumhalogeniden ist hier nicht erforderlich.

Die Erfindung wird im folgenden Anhand bevorzugter Ausführungsbeispiele näher erläutert.

Beispiel 1.

1-Butansulfonsäure

630 g (5 mol) Natriumsulfit, gelöst in 3 lWasser, wurden mit 563 g (5 mol) 1-Butylchlorid in einem 10 1-Autoklaven nach Aufdrücken von 1 bar Stickstoff bei 150 °C 8 h gerührt; der Druck im Autoklaven betrug 8 bar zum Ende der Reaktion.

Nach Beendigung der Reaktion wurde das Reaktionsgemisch zur Trockene eingedampft; Restwasser wurde durch Erwärmen im Vakuum entfernt. Man erhielt 1028 g (94%) Natrium-1-butansulfonat.

514 g (2,5 mol) des zuvor erhaltenen Natrium-1-butansulfonats wurden in 1 l Methanol suspendiert. Durch die Suspension wurde 60 Minuten lang ein Chlorwasserstoff-Strom (30 l/h) geleitet, bis 95 g (2,7 mol) Chlorwasserstoff aufgenommen waren.

Im Verlauf der Reaktion stieg die Temperatur auf 50 °C an. Anschließend wurde das ausgefallene Natriumchlorid mit 400 mol Methanol gewaschen. Aus den vereinigten methanolishcen Lösungen wurde das Methanol am Rotationsverdampfer entfernt, wobei überschüssiger Chlorwasserstoff und gegebenfalls gebildetes Schwefeldioxid ebenfalls entfernt wurden. Man erhielt 1-Butansulfonsäure als braune, leicht viskose Flüssigkeit in einer Ausbeute von 319 g (93%), bezogen auf eingesetztes 1-Butylchlorid.

Analyse:

Säurezahl: 362 (Theorie: 407)
$Na^+$-Gehalt: 0,5%
$Cl^-$-Gehalt: weniger als 0,1%
Sulfat-Gehalt: 0,8%
Wassergehalt: 4,3%

Beispiel 2.

1-Hexansulfonsäure.

96,4 g (0,8 mol) 1-Hexylchlorid wurden mit 84,4 g (0,67 mol) Natriumsulfit in 403 g Wasser 8 h lang bei 150 °C in einem Autoklaven gerührt.

Das Rohprodukt wurde zur Trockene eingedampft; der Trokkenrückstand ( 135 g ) wurde in 500 ml Methanol Suspendiert. Durch die Suspension wurde Chlorwasserstoff geleitet; die ausgefallenen Salze wurden abfiltriert und mit Methanol gewaschen. Aus den methanolischen Phasen wurde das Lösemittel abgedampft. Man erhielt 82 g 1-Hexansulfonsäure mit einer Säurezahl von 296.

Beispiel 3.

Methansulfonsäure.

278 g (5,5 mol) Methylchlorid wurden mit 630 g (5 mol) Natriumsulfit in 3 l Wasser 12 h lang in einem Autoklaven bei 100 °C gerührt.

Das Rohprodukt wurde am Rotationsverdampfer zur Trockene eingedampft; die Trockenmasse wurde in 1,4 l Methanol aufgeschlämmt. Über eine Gasfritte wurde 1 h lang Chlorwasserstoff eingeleitet, wobei die Temperatur auf 40 °C anstieg; die Chlorwasserstoff-Aufnahme betrug 325 g. Die ausgefallenen Salze wurden abfiltriert und mit Methanol gewaschen; aus den methanolischen Phasen wurde bei 70 °C/15 Torr Methanol und überschüssiger Chlorwasserstoff am Rotationsverdampfer entfernt.

Man erhielt 489 g Methansulfonsäure mit einer Säurezahl von 513.

Ansprüche

1. Verfahren zur Herstellung geradkettiger oder verzweigter $C_1$-$C_6$-Alkansulfonsäuren aus den Alkalisalzen derselben, dadurch gekennzeichnet, daß man die Alkalisalze in einer Lösung oder Suspension in $C_1$-$C_4$-Monoalkanolen mit Chlorwasserstoff umsetzt, die ausgefallenen Alkalichloride abtrennt und die $C_1$-$C_6$-Alkansulfonsäuren aus der $C_1$-$C_4$-Monoalkanolphase isoliert.

2. Verfahren nach Anspruch 1, dadurch gekennzeichnet, daß man als $C_1$-$C_4$-Monoalkanol Methanol einsetzt.

Verfahren nach Anspruch 1 oder 2, dadurch gekennzeichnet, daß man als Alkalisalze der $C_1$-$C_6$-Alkansulfonsäuren die Natriumsalze verwendet.

4. Verfahren nach Anspruch 3, dadurch gekennzeichnet, daß man die Natriumsalze der $C_1$-$C_6$-Alkansulfonsäuren in Form von Gemischen mit Natriumhalogeniden verwendet, wie sie durch Umsetzung der entsprechenden $C_1$-$C_6$-Alkylhalogenide mit Natriumsulfit in wässrigen Lösungen und Eindampfen des Reaktionsgemisches erhältlich sind.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-2 547 906 (J.K. FINCKE) <br> * Spalte 3, Zeilen 43-47 * <br> --- | 1-3 | C 07 C 143/02 |
| X | US-A-3 480 636 (B. LOEV) <br> * Spalte 1, Zeile 54 - Spalte 2, Zeile 2 * <br> --- | 1-3 | |
| A | JOURNAL OF THE AMERICAN CHEMICAL SOCIETY, Band 62, Nr. 5, 6. Mai 1940, Seite 1044, Easton, PA, US; S. ZUFFANTI: "The preparation of some branched chain aliphatic sulfonic acids" <br> * Spalte 2, Absatz 1 * <br> ----- | 1-4 | |

RECHERCHIERTE SACHGEBIETE (Int. Cl.4)

C 07 C 143/00
C 07 C 139/00

Der vorliegende Recherchenbericht wurde für alle Patentansprüche erstellt

| Recherchenort | Abschlußdatum der Recherche | Prüfer |
|---|---|---|
| DEN HAAG | 05-06-1989 | ZAROKOSTAS K. |